# EUROPEAN PATENT SPECIFICATION

(11) **EP 4 746 700 B1**
(45) Date of publication and mention of the grant of the patent: **09.12.2026**
(21) Application number: 25779837.1
(22) Date of filing: 22.09.2025
(51) Int. Cl.: A01K 67/31, B65D 65/46, B65D 85/50, A01K 67/362, A01K 67/36

(54) **TRANSPORT PACKAGING FOR FLY OFFSPRING**
TRANSPORTVERPACKUNG FÜR FLIEGENNACHWUCHS
EMBALLAGE DE TRANSPORT POUR JEUNES MOUCHES

(30) Priority: 02.10.2024 LU 103402
(43) Date of publication of application: 27.05.2026
(73) Proprietor: REPLOID GROUP AG, 4600 Wels (AT)
(72) Inventor: FINK, Jonas, 4600 Wels (AT); HEMPEL, Kai, 4600 Wels (AT); WIESNER, Moritz, 4600 Wels (AT)
(74) Representative: Tegethoff, Sebastian
(86) International application number: PCT/EP2025/077003
(87) International publication number: WO 2026/073754

(56) References cited:
- AU-A4- 2019 101 113
- KR-A- 20240 079 049
- US-A1- 2015 128 482
- US-A1- 2020 296 920

## Description

### Field of the Disclosure

The present disclosure relates to a transport packaging for fly offspring.

### Brief description of the related art

Diptera, particularly the black soldier fly, is mass-reared on organic side streams and biowaste to produce larval biomass and frass, among others used as pet and animal feed and fertilizer. In mass-rearing facilities, adult flies emerge from pupae in different containment systems such as rooms or cages and can then be utilized to produce offspring for organic side stream and biowaste recycling for high-value products.

Young fly larvae with a size of about 0.02 to 50 mg are an indispensable input material for fly larvae mass rearing facilities. Mass rearing of fly larvae based on young larvae and production of young larvae offspring can happen in two separate facilities and locations, requiring efficient and time-accurate collection, transport and delivery of young larvae from one reproduction to one or more mass rearing facilities without influencing the fitness of larvae.

The state of the art provides solutions for transporting larvae from or to mass rearing facilities. Published International patent application WO 2020/246873 A1 relates to a device for use in large-scale industrial insect farming. More in particular, the invention relates to an insects transport device for transporting live insects from a first location to a predetermined second location, the insects transport device comprising a gas guiding unit, a gas discharge member and a feeder arrangement, wherein the insects transport device is configured to receive live insects such as freshly hatched neonate larvae, for example of black soldier fly, or mites, wherein the live insects are directly taken up in a laminar flow of gas after exiting the feeder arrangement in a free fall under influence of gravitation such that the live insects do not contact any surface of the insects transport device, and while in said gas are transported to a predetermined location in the insects transport device. Furthermore, the invention relates to the use of the device in industrial insect farming, such as large-scale farming of black soldier flies or mites. The invention also relates to a method of dosing live insects, wherein for example live insects are dosed which are essentially of the same age (e.g. within an age difference of 1 second - 5 minutes), such as freshly hatched neonate larvae. In addition, the invention relates to a single dose of neonate larvae obtained with the method, wherein larvae have a small larvae-to-larvae age difference within the single dose.

Published International patent application WO 2023/118335 A2 relates to a mobile transport device for transporting insect larvae, comprising a housing with a selectively openable and closable opening, a receiving section arranged within the housing for receiving at least one first insect fattening container, wherein the first insect fattening container is received in the receiving portion and adapted to receive a first insect larvae cohort for fattening, a recirculation fan for partially recirculating air within the housing, an electronic control unit for controlling the recirculation fan, and a first air regulating device, which has a first ventilation section on a first side and a first venting section on a second side, and wherein air enters the first insect fattening container through the first ventilation section and exits the first insect fattening container through the first venting section under the mediation of the recirculation fan. The invention further relates to a method and a use.

Published European patent application EP 3 309 423 A1 discloses a transportable container for the breeding of invertebrates with a three-dimensional prismatic interior of gyroid structure of continuous development characterized by being made up of a container of straight rectangular parallelepiped shape which has a top cover in its upper part and two rings in the middle of its side faces, and where in the lower third of the container's height there is a perimetral elastic element to be able to help the assembly vibrate without causing breakage; bellow the perimetral elastic element and in the lateral faces there are two couplings to fix a hose; at the outer side of the container there is a sliding profile, which is located on the inside of the container and is positioned on the inner side of the container; there is a waste collection tray that runs along the surface of the profile, which has two semi-circular tabs to allow the removal of the tray; at the bottom of the container there are four legs made up of two L-shaped plates that start from the four corners of the container and rest on a transport platform carried by a mobile vehicle; and there is a central element that occupies 70% of the container formed The central element that occupies 70% of the container is a three-dimensional prismatic interior of gyroid structure of continuous development, built by a 3D printer in plastic material, where the internal or empty part of this body is over 98% of it.

Published International patent application WO 2021/186428 A1 provides compositions and methods for inducing suspended animation in neonate larvae of beneficial fly, particularly larvae of the black soldier fly. During the suspended animation, the larvae show extended shelf-life and improved shipment durability. The suspended animation is reversible, and upon exposure to standard rearing conditions the larvae reach comparable maturity to corresponding non- suspended larvae.

Published European patent application EP 4 523 526 A1 discloses a package to improve insect breeding, in particular to increase the accuracy of the dosage of insects in the container, for example depending on a substrate in the container, and thus to improve the growth of the insects in the container and the breeding success. The packaging is filled with a predetermined quantity of insects, wherein the insects are at a specific growth stage, in particular as larvae, or at different specific growth stages, wherein the packaging is closed to prevent the insects from escaping from the packaging, wherein the packaging is designed to open at least partially under at least one predetermined environmental condition and/or through consumption by the insects in the packaging in order to release the insects.

Published Korean patent application KR 2024 0079049 A discloses a feed package for insect breeding may include a case having an internal space and a feed layer accommodated in the internal space. The feed layer may include a first feed layer including at least one of a first mealworm molting shell, a first agricultural by-product, a first fishery by-product, and a first mainstream by-product, a moisture supply layer formed on the first feed layer and having a moisture content of 30 wt% or more, and a second feed layer formed on the moisture supply layer and including at least one of a second mealworm molting shell, a second agricultural by-product, a second fishery by-product, and a second mainstream by-product.

Published Australian patent application AU 2019 101 113 A4 discloses capsules, systems and methods for delivering parasitic insects and/or predatory insects to agricultural crops. In particular, the parasitic insects are parasitic wasps and the predatory insects are predatory mites and are contained in delivery capsules, which may degrade in the environment. The capsules comprise first orifices sized so that an insect cannot exit the capsules and second orifices sized so that an insect can exit the capsules. By creating the second orifices shortly before capsule delivery the number of insects reaching agricultural crops is increased.

Published U.S. application US 2015/128482 A1 discloses enclosures for mass delivery of a living biological pest control agent according to the embodiments disclosed herein include a body section having a generally cylindrical wall with opposed ends defining an interior surface, parasitized eggs of a living biological pest control agent (e.g., an endoparasitic insect) adhered to the interior surface of the wall, and at least one egress opening to allow the pest control agent to exit the body section to an exterior environment.

Published U.S. patent application US 2020/296920 A1 relates to an insect storage and release device includes suitably rigid container that may be used to store and transport insects. The insect storage and release device may hold compressed insects or non-compressed insects. The insect storage and release device includes a removable enclosure and a reticulated foam for adding compliance during packing of the insects.

None of the known solution is in standard sizes for currently used transport infrastructure like pallets. The existing solutions do further not sufficiently reduce the overall volume to be shipped making transport expensive. In addition, the known solutions require returning the transport device to the place of larvae offspring production with complex and expensive logistics, in addition to cleaning and handling of transport devices before re-use. Finally, the known solutions for transporting insect larvae will have to be unpacked before use when reaching their destination which causes additional effort.

Thus, there is a need for a packaging for fly larvae avoiding the disadvantages of the known solutions.

### Summary of the Disclosure

The present disclosure provides a packaging made of a biodegradable polymer, comprising fly offspring.

The packaging is water dissolvable, has a volume between 0.2 to 60 1 and comprises between 6,500 to 300,00 fly offspring.

The fly offspring are selected from larvae, pre-pupae or pupae, wherein in an embodiment, the fly offspring are black soldier fly offspring.
It is intended that the packaging is a bag or capsule, wherein the packaging has in an embodiment a volume between 200 to 1200 ml and comprises between 100,000 to 300,000 fly offspring, in another embodiment a volume between 200 to 4800 ml and comprises between 25,000 to 120,000 fly offspring, and in another embodiment a volume of 200 to 1200 ml and comprises between 6,500 to 30,000 fly offspring.

The disclosure comprises an embodiment of a packaging additionally comprising residual feed substrate or frass.

An aspect of the disclosure relates to a packaging comprising larvae and wherein the packaging has a volume between 1.5 to 60 1 and comprises between 100,000 to 300,00 larvae. Another aspect relates to a packaging having a volume between 0.2 to 24 l and comprising between 25,000 to 120,000 larvae, or a packaging having a volume between 0.2 to 6 l and comprising between 6,500 to 30,000 larvae.

It is intended that the packaging is made of polyvinyl alcohol (PVA), polyvinyl alcohol (PVOH), alginate, pullulan, polylactide, xanthan, gummi arabicum, polylactic Acid (PLA), starch-based biopolymers derived from corn or potatoes, gelatin, carboxymethyl cellulose (CMC), or other water-soluble biopolymers

Another object of the present disclosure relates to a method for transporting fly offspring comprising the steps of:
- Filling fly offspring into a packaging according to any one of claims 1 to 12 made of a biodegradable polymer;
- Sealing of packaging;
- Transporting the fly offspring in the sealed packaging.

Another object of the present disclosure relates to a method for rearing fly offspring, comprising the steps of:
- Filling fly offspring into a water-dissolvable packaging made of a biodegradable polymer;
- Sealing of packaging;
- Transporting the fly offspring in the sealed packaging; and
- Proving the packaging to a medium for rearing of the fly offspring, wherein the medium comprises water for dissolving the packaging.

The foregoing summary is not intended to summarize each potential embodiment or every aspect of the present disclosure.

### Summary of the Figures

The disclosure will be described based on figures. It will be understood that the embodiments and aspects of the disclosure described in the figures are only examples and do not limit the protective scope of the claims in any way. The disclosure is defined by the claims and their equivalents. It will be understood that features of one aspect or embodiment of the disclosure can be combined with a feature of a different aspect or aspects of other embodiments of the disclosure, in which:

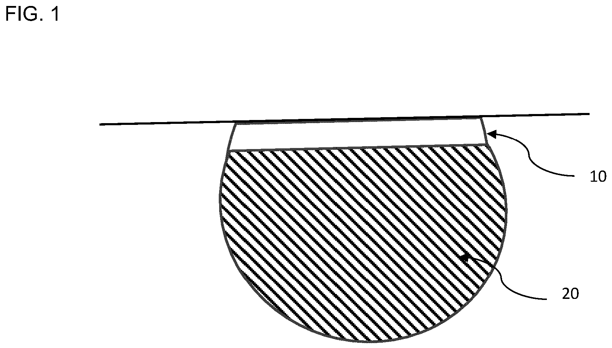

FIG. 1 shows schematically an example of a packaging according to the present disclosure.

### Detailed Description of the Disclosure and the Figures

The technical problem is solved by the independent claims. The dependent claims cover further specific embodiments of the disclosure.

The present disclosure provides a packaging for fly offspring made of a bio-dissolvable plastic. The term fly offspring relates to larvae, pre-pupae, and pupae of flies. The dissolvable packaging consisted of materials such as polyvinyl alcohol (PVA), polyvinyl alcohol (PVOH), alginate, pullulan, polylactid, xanthan, gummi arabicum, polylactic Acid (PLA), starch-based biopolymers (e.g., corn, potatoes), gelatin, carboxymethyl cellulose (CMC), or other similar water-soluble biopolymers.

The present disclosure further discloses a method for packaging and transporting fly offspring and providing them in the packaging for their rearing on a medium or organic side streams and biowaste without requiring their unpacking.

For the purposes of the present disclosure, the term biodegradable should be understood to mean a property of polymers or polymer mixtures that they can be biodegraded in or on an aqueous medium without causing any harm to humans or animals. The term frass relates in the present context to the excrement and discarded organic matter produced by insects, in particular during their development.

FIG. 1 shows schematically an example of a packaging 10 according to the present disclosure comprising fly offspring and/or frass and/or residual substrate 20. The packaging is made of a polymer or polymer mixture which is biodegradable in an aqueous environment. Thus, the material of the packaging is water-dissolvable.

The packaging can be a bag or a capsule with different sizes. It is envisaged that the packaging has a volume of 200 to 1200 ml comprising 6,500 to 30,000 fly offspring. Another embodiment of the packaging relates to a volume of 200 to 4800 ml comprising 25,000 to 120,000 fly offspring. Finally, the packaging has a volume of 200 to 1200 ml comprising 100,000 to 300,000 fly offspring. The fly offspring have a weight of about 0.015 to 200 mg per organism.

It is also intended that the packaging comprises fly offspring and residual feed substrate or frass. In this embodiment, the packaging has a volume of 0.2 to 6 l comprising 6,500 to 30,000 fly offspring, 0.2 to 24 l comprising 25,000 to 120,000 fly offspring or 1.5 to 60 l comprising 100,000 to 300,000 fly offspring.

The packed fly offspring will be transported in a packaging according to the present disclosure from a reproduction facility to a bioconversion facility. The packaging is stable during transport and dissolves in or on a substrate for rearing of the fly offspring. The biodegradation of the packaging is initiated by water which is comprised in the substrate for fly rearing.

Advantages of the subject matter of the present disclosure relate to providing a packaging which allows a packing of fly offspring in standard boxes which are used for a transport reducing packing, collection and transport costs. The provided packaging is stable during its transport and there is no reduction of the fitness of the fly offspring.

Another advantage related to the subject matter of the present disclosure is that no unpackaging is required because the packaging is biodegradable or consumed by the fly offspring during bioconversion in an appropriate environment.

Another advantage of the subject matter is that the transport device dissolved in water and consequently no transport of the transport device back to the reproduction location, in addition to handling and cleaning of the transport device is needed, reducing logistical complexities and costs.

The foregoing description of preferred and other embodiments is not intended to limit or restrict the scope or applicability of the inventive concepts conceived of by the Applicants. It will be appreciated with the benefit of the present disclosure that features described above in accordance with any embodiment or aspect of the disclosed subject matter can be utilized, either alone or in combination, with any other described feature, in any other embodiment or aspect of the disclosed subject matter.

### Reference Numerals

- 10: packaging
- 20: fly offspring / frass / residual substrate

## Claims

1. A packaging made of a biodegradable polymer, wherein the packaging is water-dissolvable, **characterised in that** the packaging and has a volume between 0.2 to 60 l and comprises between 6,500 to 300,000 fly offspring.

2. The packaging of claim 1, wherein the fly offspring are selected from larvae, pre-pupae or pupae.

3. The packaging of claim 2 , wherein the fly offspring are black soldier fly offspring.

4. The packaging of any one of claims 1 to 3, wherein the packaging is a bag or capsule.

5. The packaging of any one of claims 1 to 4, wherein the packaging has a volume between 200 to 1200 ml and comprises between 100,000 to 300,000 fly offspring.

6. The packaging of any one of claims 1 to 5, wherein the packaging has a volume between 200 to 4800 ml and comprises between 25,000 to 120,000 fly offspring.

7. The packaging of any one of claims 1 to 6, wherein the packaging has a volume of 0.2 to 1200 ml and comprises between 6,500 to 30,000 fly offspring.

8. The packaging of any one of claims 1 to 7, wherein the packaging is made of polyvinyl alcohol (PVA), polyvinyl alcohol (PVOH), alginate, pullulan, polylactide, xanthan, gummi arabicum, polylactic Acid (PLA), starch-based biopolymers derived from corn or potatoes, gelatin, or carboxymethyl cellulose (CMC.

9. The packaging of any one of claims 1 to 8, comprising additionally residual feed substrate or frass.

10. The packaging of claim 9, wherein the packaging comprises larvae and wherein the packaging has a volume between 1.5 to 60 1 and comprises between 100,000 to 300,00 larvae and the residual feed substrate or frass.

11. The packaging of claim 9 or 10, wherein the packaging has a volume between 0.2 to 24 l and comprises between 25,000 to 120,000 larvae and the residual feed substrate or frass.

12. The packaging of any one of claims 9 to 11, wherein the packaging has a volume between 0.2 to 6 l and comprises between 6,500 to 30,000 larvae and the residual feed substrate or frass.

13. A method for transporting fly offspring comprising the steps of:
- Providing a packaging according to any one of claims 1 to 12;
- Sealing of packaging;
- Transporting the fly offspring in the sealed packaging.

14. A method for rearing fly offspring, comprising the steps of:
- Filling fly offspring into a water-dissolvable packaging made of a biodegradable polymer;
- Sealing of packaging;
- Transporting the fly offspring in the sealed packaging; and
- Proving the packaging to a medium for rearing of the fly offspring, wherein the medium comprises water for dissolving the packaging.

## Patentansprüche

1. Eine Verpackung aus einem biologisch abbaubaren Polymer, wobei die Verpackung wasserlöslich ist, **dadurch gekennzeichnet, dass** die Verpackung ein Volumen zwischen 0,2 und 60 l aufweist und zwischen 6.500 und 300.000 Fliegennachkommen enthält.

2. Die Verpackung nach einem der Ansprüche 1, wobei die Fliegennachkommen aus Larven, Vorpuppen oder Puppen ausgewählt sind.

3. Die Verpackung nach Anspruch 2, wobei es sich bei den Fliegennachkommen um Nachkommen der Schwarzen Soldatenfliege handelt.

4. Die Verpackung nach einem der Ansprüche 1 bis 3, wobei die Verpackung ein Beutel oder eine Kapsel ist.

5. Die Verpackung nach einem der Ansprüche 1 bis 4, wobei die Verpackung ein Volumen zwischen 200 und 1.200 ml aufweist und zwischen 100.000 und 300.000 Fliegennachkommen enthält.

6. Die Verpackung nach einem der Ansprüche 1 bis 5, wobei die Verpackung ein Volumen zwischen 200 und 4800 ml aufweist und zwischen 25.000 und 120.000 Fliegennachkommen enthält.

7. Die Verpackung nach einem der Ansprüche 1 bis 6, wobei die Verpackung ein Volumen von 0,2 bis 1200 ml aufweist und zwischen 6.500 und 30.000 Fliegennachkommen enthält.

8. Die Verpackung nach einem der Ansprüche 1 bis 7, wobei die Verpackung aus Polyvinylalkohol (PVA), Polyvinylalkohol (PVOH), Alginat, Pullulan, Polylactid, Xanthan, Gummi arabicum, Polymilchsäure (PLA), aus Mais oder Kartoffeln gewonnenen Biopolymeren auf Stärkebasis, Gelatine oder Carboxymethylcellulose (CMC) besteht.

9. Die Verpackung nach einem der Ansprüche 1 bis 8, die zusätzlich Restfuttersubstrat oder Frass enthält.

10. Die Verpackung nach Anspruch 9, wobei die Verpackung Larven enthält und wobei die Verpackung ein Volumen zwischen 1,5 und 60 l aufweist und zwischen 100.000 und 300.000 Larven sowie das restliche Futtersubstrat oder den Frass enthält.

11. Die Verpackung nach Anspruch 9 oder 10, wobei die Verpackung ein Volumen zwischen 0,2 und 24 l aufweist und zwischen 25.000 und 120.000 Larven sowie das restliche Futtersubstrat oder den Frass enthält.

12. Die Verpackung nach einem der Ansprüche 9 bis 11, wobei die Verpackung ein Volumen zwischen 0,2 und 6 l aufweist und zwischen 6.500 und 30.000 Larven sowie das verbleibende Futtersubstrat oder den Frass enthält.

13. Ein Verfahren zum Transport von Fliegennachkommen, umfassend die folgenden Schritte:
- Bereitstellen einer Verpackung gemäß einem der Ansprüche 1 bis 12;
- Versiegeln der Verpackung;
- Transportieren der Fliegennachkommen in der versiegelten Verpackung.

14. Ein Verfahren zur Aufzucht von Fliegennachkommen, umfassend die folgenden Schritte:
- Einfüllen der Fliegennachkommen in eine wasserlösliche Verpackung aus einem biologisch abbaubaren Polymer;
- Verschließen der Verpackung;
- Transport der Fliegenlarven in der versiegelten Verpackung; und
- Bereitstellen der Verpackung in einem Medium zur Aufzucht der Fliegennachkommen, wobei das Medium Wasser zum Auflösen der Verpackung umfasst.

## Revendications

1. Un emballage constitué d'un polymère biodégradable, ledit emballage étant soluble dans l'eau, **caractérisé en ce que** l'emballage a un volume compris entre 0,2 et 60 l et contient entre 6 500 et 300 000 larves de mouches.

2. L'emballage selon l'une quelconque des revendications 1, dans lequel les descendants de mouches sont choisis parmi des larves, des pré-pupes ou des pupes.

3. L'emballage selon la revendication 2, dans lequel les descendants de mouches sont des descendants de la mouche soldat noire.

4. L'emballage selon l'une quelconque des revendications 1 à 3, dans lequel l'emballage est un sachet ou une capsule.

5. L'emballage selon l'une quelconque des revendications 1 à 4, dans lequel l'emballage a un volume compris entre 200 et 1 200 ml et contient entre 100 000 et 300 000 descendants de mouches.

6. L'emballage selon l'une quelconque des revendications 1 à 5, dans lequel l'emballage a un volume compris entre 200 et 4 800 ml et contient entre 25 000 et 120 000 descendants de mouches.

7. L'emballage selon l'une quelconque des revendications 1 à 6, dans lequel l'emballage a un volume compris entre 0,2 et 1 200 ml et contient entre 6 500 et 30 000 descendants de mouches.

8. L'emballage selon l'une quelconque des revendications 1 à 7, **caractérisé en ce que** l'emballage est constitué d'alcool polyvinylique (PVA), d'alcool polyvinylique hydroxylé (PVOH), d'alginate, de pullulane, de polylactide, de gomme xanthane, de gomme arabique, d'acide polylactique (PLA), de biopolymères à base d'amidon dérivés du maïs ou de la pomme de terre, de gélatine ou de carboxyméthylcellulose (CMC).

9. L'emballage selon l'une quelconque des revendications 1 à 8, comprenant en outre des résidus de substrat alimentaire ou des excréments.

10. L'emballage selon la revendication 9, dans lequel l'emballage comprend des larves et dans lequel l'emballage a un volume compris entre 1,5 et 60 l et comprend entre 100 000 et 300 000 larves ainsi que le substrat alimentaire résiduel ou les excréments.

11. L'emballage selon la revendication 9 ou 10, dans lequel l'emballage a un volume compris entre 0,2 et 24 l et contient entre 25 000 et 120 000 larves ainsi que le substrat alimentaire résiduel ou les excréments.

12. L'emballage selon l'une quelconque des revendications 9 à 11, **caractérisé en ce que** l'emballage a un volume compris entre 0,2 et 6 l et contient entre 6 500 et 30 000 larves ainsi que le substrat alimentaire résiduel ou les excréments.

13. Un procédé de transport de la progéniture de mouches, comprenant les étapes suivantes:
- fournir un emballage selon l'une quelconque des revendications 1 à 12 ;
- fermer hermétiquement l'emballage ;
- le transport des descendants de mouches dans l'emballage scellé.

14. Un procédé d'élevage de la progéniture de mouches, comprenant les étapes suivantes :
- Introduire la progéniture de mouches dans un emballage hydrosoluble constitué d'un polymère biodégradable ;
- fermer hermétiquement l'emballage ;
- le transport des larves de mouches dans l'emballage scellé ; et
- Placer l'emballage dans un milieu destiné à l'élevage des larves de mouches, ledit milieu comprenant de l'eau permettant de dissoudre l'emballage.
